Europäisches Patentamt

(19) European Patent Office

Office européen des brevets

(11) Publication number: **0 378 296**
**A2**

# EUROPEAN PATENT APPLICATION

(21) Application number: **90300059.4**

(22) Date of filing: **03.01.90**

(51) Int. Cl.⁵: **A61M 1/00**

(30) Priority: **12.01.89 GB 8900623**

(43) Date of publication of application:
**18.07.90 Bulletin 90/29**

(84) Designated Contracting States:
**DE DK ES FR IT**

(71) Applicant: **Smiths Industries Public Limited Company**
**765, Finchley Road**
**London, NW11 8DS(GB)**

(72) Inventor: **Hannant, Keith**
**43, Cove Road**
**Rustington West Sussex BN16 2QN(GB)**
Inventor: **Hart, Barrie**
**1, Almond Hayes**
**Ipswich 1P2 9SH(GB)**
Inventor: **Wood, James Michael**
**5, The Grovelands**
**Lancing West Sussex BN15 8HY(GB)**

(74) Representative: **Flint, Jonathan McNeill**
**SMITHS INDUSTRIES PUBLIC LIMITED**
**COMPANY 765 Finchley Road**
**London NW11 8DS(GB)**

(54) Suction pump assemblies.

(57) A medical suction pump has a fluid collection jar 1 and a diaphragm pump 2. The pump 2 has a lower rigid plate 20 that is sealed at its edge to the jar, and an upper flexible plate 31 which is coupled to a drive unit 40 and which can be detached from the lower plate so that the lower plate and jar 1 can be disposed of, whilst the upper plate is retained with the drive unit. In one arrangement, the pump 2 has one-way valves 27 and 36 which enable it to create a reduced gas pressure in the jar so that fluid is drawn into the jar without passing through the pump chamber 39. In another arrangement, the fluid is pumped through the pump chamber (39'). In this arrangement the upper plate (31') is preferably disposed of with the lower plate and the suction jar which could be a flexible bag (1').

*Fig. 1.*

## SUCTION PUMP ASSEMBLIES

This invention relates to suction pump assemblies of the kind including a fluid collection container and a pump coupled with the container for drawing fluid into the container for collection therein and subsequent disposal.

The invention is more particularly concerned with suction pump assemblies for medico-surgical or other applications where hazardous fluids are collected.

In conventional medico-surgical suction pump assemblies, a pump is interconnected via tubing to an outlet provided near the top of a collection jar. An inlet to the collection jar is connected via tubing to a suction catheter which the surgeon applies to the surgical site to remove blood, aspirating fluid, tissue debris or the like. The pump draws air out of the collection jar, creating a reduced pressure which causes the fluid to be sucked along the catheter tubing into the jar. When the collection jar is full, it is disconnected from the pump and disposed of. Preferably, some form of bacterial filter is connected between the collection jar and the pump to prevent contamination of the pump and atmosphere by bacteria. Although these filters provide a degree of protection, it is nevertheless necessary periodically to clean and sterilize the pump which is a difficult and time consuming task. There is risk that a pump infected by bacteria may be used for some time before it is next sterilized since this is not generally done after every use.

It is an object of the present invention to provide a suction pump assembly that can be used to overcome these disadvantages.

According to one aspect of the present invention there is provided a suction pump assembly of the above-specified kind characterised in that the pump includes a first substantially rigid plate secured around its edge to the container and a one-way valve establishing fluid communication between the container and the pump, a second flexible diaphragm plate sealed arround its edge with the first plate and defining between the first and second plates a pump chamber of variable volume, and a drive unit for applying osciallatory movement to the second plate so as to vary the volume of the pump chamber and thereby cause fluid to be drawn into the container, at least the container and first plate being readily removable from the drive unit so that the container and its contents can be removed and disposed of, with the first plate providing a cover for the container.

In one arrangement, the pump creates a reduced air pressure in the container so that fluid is drawn into the container through a passage not including the pump chamber, the one-way valve allowing gas out of the container but preventing gas entering the container, and the second plate including a one-way valve that allows gas to leave the pump chamber but prevents gas entering the pump chamber.

The first plate preferably has a vent tube which extends to the one-way valve and through which gas is withdrawn from the container, the lower end of the vent tube defining the maximum filling level of the container. The assembly may include a filter which is permeable to gas but impermeable to liquid, the filter being in line with the one-way valve. The pump may include a fluid inlet spigot formed with the first plate.

In an alternative arrangement, suction fluid flows into the container through the pump chamber, and the one-way valve allows fluid to flow into the container from the pump chamber but prevents flow in the opposite direction. In this alternative arrangement, the container may be a flexible bag.

The first plate may be detachable from the second plate, the second plate being retained with the drive unit and the first plate being disposed of with the container.

The assembly preferably includes an outer housing within which the container is located, the outer housing including a clip that holds the pump on the housing and holds the first and second plates together.

The second plate may alternatively be permanently secured with the first plate such that the second plate is disposed of with the first plate.

Two forms of suction pump assemblies, in accordance with the present invention, will now be described, by way of example, with reference to the accompanying drawings, in which:

Figure 1 is a sectional side elevation view of one form of assembly; and

Figure 2 is a sectional side elevation view of the other form of assembly.

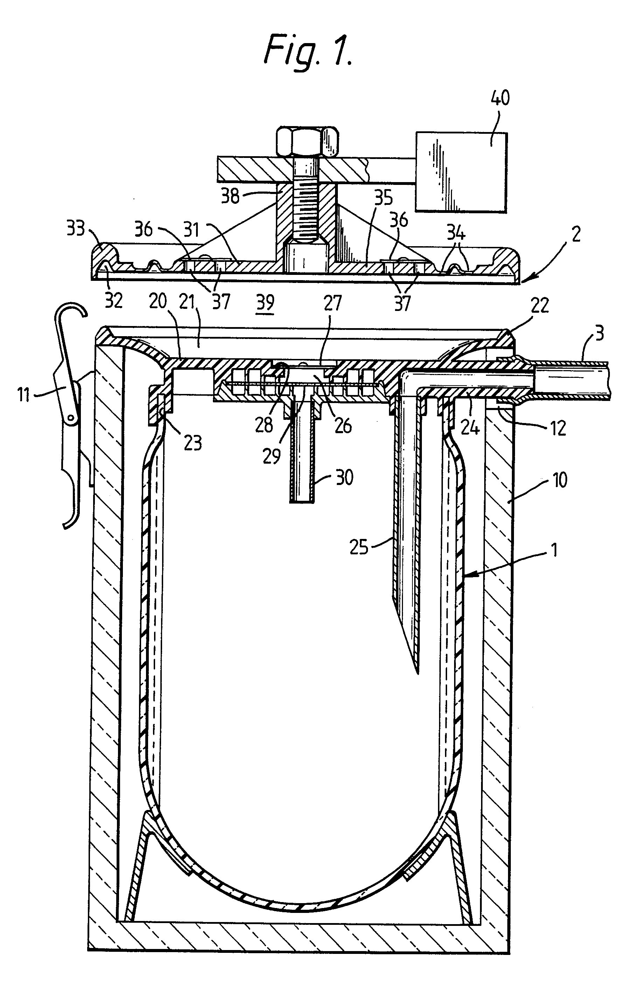

With reference to Figure 1, the suction pump assembly comprises a collection jar 1 and a diaphragm pump 2 which creates a reduced pressure in the jar to draw fluid into the jar from a suction catheter, via tubing 3.

The jar 1 is of circular section with a capacity of two litres and is preferably moulded from an inexpensive, transparent plastics material so that it can be disposed of when full. The jar 1 is supported within an outer housing 10 of a rigid material. The outer housing 10 is also preferably transparent, or has a transparent window, so that the filling level of the jar 1 can be monitored. At its upper end, toggle fasteners 11, only one of which is shown, are mounted on the outer surface of the housing which serve to secure the jar 1 and the diaphragm pump 2 to the housing 10.

The pump 2 has a rigid lower plate member 20 moulded from a plastics material. The plate 20 is of generally dish shape with a shallow concave recess 21 on its upper surface. The lower surface of the outer edge of the plate 20 rests on the top of the housing 10, the upper surface of the plate having an upwardly projecting wall 22 around its edge. The plate 20 is sealed to the top of the jar 1 by means of a downwardly projecting annular channel 23 which receives the top edge of the jar and is permanently secured to it, such as by means of an adhesive or by welding. At one edge of the plate member 20, a spigot 24 projects radially outwardly beneath the edge of the plate and extends through a keyway 12 in the upper edge of the housing 10. The spigot 24 defines a fluid passageway through the plate 20 which opens into the jar 1 via a vertical inlet tube 25.

A gas passage 26 extends into the jar 1 through the centre of the plate member 20. The passage 26 is sealed by a one-way valve 27 in the form of a conventional resilient flap-valve the edge on which seals on an annular shoulder 28 formed around the passage 26. The valve 27 allows upward flow of gas, that is, from the jar 1 into the pump 2, but prevents flow in the opposite direction. Beneath the valve 27 there is mounted a hydrophobic bacterial filter 29 which communicates with a short gas vent tube 30. The filter allow passage of gas but prevents passage of bacteria or liquids. Other gas-permeable, liquid-impermeable filters could be used.

The pump 2 also includes an upper plate member 31 which is made of a resilient, elastomeric material such as rubber or an elastomeric plastics. The upper plate 31 is generally flat in its natural state and of circular shape A concave annular channel 32 extends around the lower surface of the outer edge of the plate 31, the channel receiving the wall 22 on the lower plate 20 in sealing engagement. Around the edge of the upper surface of the upper plate 31, there is an annular wall 33 with a curved top which is engaged by the toggle fasteners 11 to hold the upper plate securely on the lower plate and to clamp the pump 2 on the housing 1. A short distance within the edge of the upper plate 31, it is reduced in thickness and formed into two annular concentric corrugations 34. The central region 35 of the upper plate 31 is thicker and stiffer and supports two one-way valves 36 that overlie gas vents 37 through the plate. The valves 37 are conventional resilient flap valves which are arranged to allow gas to flow up through the vents 37 and out of the pump 2 but to prevent gas flow in the opposite direction. In the centre of the upper plate 31, there projects upwardly a vertical mounting stem 38 by which the pump is coupled to a drive unit 40.

The drive unit 40 is a conventional oscillatory motor which is coupled to the stem 38 and moves this up and down, thereby moving the central region 35 of the upper plate 31 up and down and flexing the corrugations 34. This varies the volume of the pump chamber 39 which is defined between the upper plate 31 and the lower plate 20.

In operation, the pump 2 and jar 1 are clamped firmly onto the housing 10 by means of the toggle fasteners 11. The drive unit 40 is then energized so that the central region 35 of the upper plate 31 is moved up and down, away from and towards the lower plate 20. Downward movement of the upper plate 31 causes an increase in pressure in the pump chamber 39 thereby closing the valve 27 and opening the valves 36 which allows air to escape from the chamber to atmosphere. Upward movement of the upper plate 31 causes a reduction of pressure within the chamber 39 thereby closing the valves 36 but opening the valve 27 into the jar. This causes gas in the jar 1 to be drawn through the vent tube 30 and filter 29 into the pump chamber 39. As this is repeated, it can be seen that gas will be pumped out of the jar 1, causing a reduced pressure in the jar and thereby causing suction to be applied to the suction catheter 3. Fluid from the surgical site will enter the jar via a passage including the catheter 3 and the inlet tube 25 but not including the pump chamber 39, gradually filling the jar.

When the desired filling level is reached, which is somewhere below the bottom of the vent tube 30, the user turns off the pump drive unit 40. If the fluid level should rise too far, and it enters the vent tube 30, the filter 29 will prevent it passing into the pump 2. The toggle fasteners 11 are now released and the jar 1, with the lower plate 20, is removed from the housing 10. The lower plate 20 acts as a cover for the jar 1 to prevent spillage, although preferably an additional cover will be used or some form of plug to occlude the spigot 24. The jar 1 and plate 20 are then disposed of and a new jar with its own lower plate are placed in the housing 10. The upper plate 31 is reused and clamped onto the lower plate using the toggle fasteners 11.

Suction pump assemblies of the present invention avoid the need for any tubing between the collection jar and pump. Although the upper plate 31 may need cleaning occasionally, because of its open construction this can be readily performed, such as in an autoclave.

The assembly described above creates a reduced pressure in the collection jar by pumping out gas from the jar. This requires the use of a jar that is rigid and can withstand the reduced pressures employed.

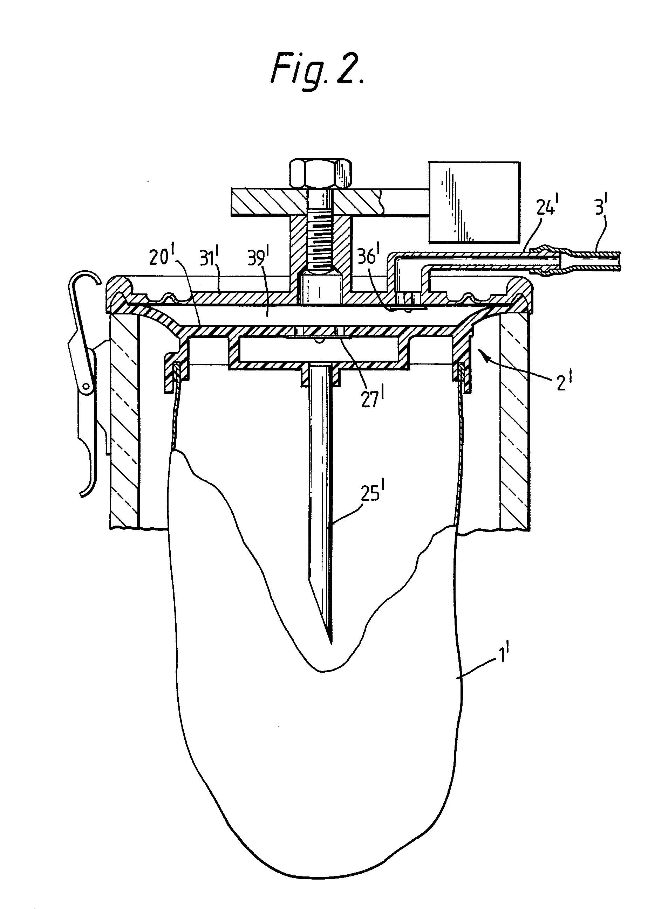

With reference now to Figure 2, there is shown an alternative suction pump assembly similar to that of Figure 1 but which is used directly to pump suction fluid into the container 1. The inlet spigot 24′ in this arrangement opens into the pump chamber 39′ via a one way valve 36′ which allows fluid into the pump chamber but prevents it leaving the chamber. The pump chamber 39′ communicates with the container 1′, which in this case is a flexible bag, via an inlet tube 25′ and a one-way valve 27′. The one-way valve 27′ allows fluid to flow from the pump chamber 39′ to the inlet tube 25′ but prevents flow in the opposite direction.

When the upper plate 31′ is raised, this creates a reduced pressure in the chamber 39′ which closes valve 27′ and draws fluid from the inlet spigot 24′ and catheter 3′ into the chamber. Downward movement of the upper plate 31′ closes valve 36′ and pumps the fluid in chamber 39′ via the valve 27′ and inlet tube 25′ into the container 1′. If the container 1′ was rigid, an air vent may be necessary to allow air to escape from the container as it fills with fluid. With this assembly, it is preferable that the upper plate 31′ is permanently sealed around its outer edge to the lower plate 20′, so that the entire pump 2′ is disposed of with the container 1′, further to avoid the risk of contamination.

In such an arrangement, because it does not create a reduced pressure in the container, the container could be a flexible bag. It will be appreciated that such an assembly would be impractical with conventional, reusable pumps because of the need to sterilise the pump after each use. In the present invention, the entire would be disposed of and only the drive unit and support housing retained.

## Claims

1. A suction pump assembly including a fluid collection container and a pump coupled with the container for drawing fluid into the container for collection therein and subsequent disposal, characterised in that the pump (2, 2′) includes a first substantially rigid plate (20, 20′) secured around its edge to the container (1, 1′) and a one-way valve (27, 27′) establishing fluid communication between the container (1, 1′) and the pump (2, 2′), a second flexible diaphragm plate (31, 31′) sealed around its edge with the first plate (20, 20′) and defining between the first and second plates a pump chamber (39, 39′) of variable volume, and a drive unit (40) for applying oscillatory movement to the second plate (31, 31′) so as to vary the volume of the pump chamber (39, 39′) and thereby cause fluid to be drawn into the container, at least the container (1, 1′) and first plate (20, 20′) being readily removable from the drive unit (40) so that the container and its contents can be removed and disposed of, with the first plate (20, 20′) providing a cover for the container.

2. A suction pump assembly according to Claim 1, characterised in that the pump (2) creates a reduced gas pressure in the container (1) so that fluid is drawn into the container through a passage (24, 25) not including the pump chamber (39), that the one-way valve (27) allows gas out of the container (1) but prevents gas entering the container, and that the second plate (31) includes a one-way valve (36, 37) that allows gas to leave the pump chamber (39) but prevents gas entering the pump chamber.

3. A suction pump assembly according to Claim 2, characterised in that the first plate (20) has a vent tube (30) which extends to the one-way valve (27) and through which gas is withdrawn from the container (1), and that the lower end of the vent tube (30) defines the maximum filling level of the container.

4. A suction pump assembly according to Claim 2 or 3, characterised in that the assembly includes a filter (29) which is permeable to gas but impermeable to liquid, and that the filter (29) is in line with the one-way valve (27).

5. A suction pump assembly according to any one of Claims 2 to 4, characterised in that the pump (2) includes a fluid inlet spigot (24) formed with the first plate (20).

6. A suction pump assembly according to Claim 1, characterised in that suction fluid flows into the container (1′) through the pump chamber (39′), and that the one-way valve (27′) allows fluid to flow into the container (1′) from the pump chamber (39′) but prevents flow in the opposite direction.

7. A suction pump assembly according to Claim 6, characterised that the container is a flexible bag (1′).

8. A suction pump assembly according to any one of the preceding claims, characterised in that the first plate (20, 20′) is detachable from the second plate (31, 31′), and that the second plate is retained with the drive unit (40) and the first plate is disposed of with the container (1,1′).

9. A suction pump assembly according to any one of the preceding claims, characterised in that the assembly includes an outer housing (10) within which the container (1,1′) is located, and that the outer housing includes a clip (11) that holds the pump (2, 2′) on the housing and holds the first and second plates (20, 20′ and 31, 31′) together.

10. A suction pump assembly according to any one of Claims 1 to 7, characterised in that the second plate (31, 31′) is permanently secured with the first plate (20, 20′) such that the second plate is disposed of with the first plate.

# Fig. 1.

# Fig.2.